# EUROPÄISCHE PATENTSCHRIFT

(11) **EP 1 952 837 B1**
(45) Veröffentlichungstag und Bekanntmachung des Hinweises auf die Patenterteilung: **20.10.2010**
(21) Anmeldenummer: 07101408.8
(22) Anmeldetag: 30.01.2007
(51) Int. Cl.: A61M 5/178, A61J 1/00, A61M 5/36

(54) **Vorrichtung zum Umfüllen einer Substanz**
Device for siphoning a substance
Dispositif destiné au transvasement d'une substance

(43) Veröffentlichungstag der Anmeldung: 06.08.2008
(73) Patentinhaber: F.HOFFMANN-LA ROCHE AG, 4070 Basel (CH); Roche Diagnostics GmbH, 68305 Mannheim (DE)
(72) Erfinder: Remde, Axel, 3432, Lützelflüh-Goldbach (CH); Zihlmann, Rudolf, 3550, Langnau (CH)
(74) Vertreter: Schwabe - Sandmair - Marx

(56) Entgegenhaltungen:
- EP-A1- 0 787 503
- WO-A-02/102295
- WO-A-03/090822
- WO-A2-2004/032997
- FR-A1- 2 762 990
- US-A- 4 338 980
- US-A- 6 162 199
- US-B1- 6 439 276

## Beschreibung

Die vorliegende Erfindung betrifft eine Vorrichtung zum Umfüllen einer Substanz und insbesondere eines Arzneimittels, wie zum Beispiel Insulin oder Hormone, um zum Beispiel eine in einer Pumpe verwendbare Ampulle aus einer gekauften Ampulle oder aus einem anderen das Arzneimittel enthaltende Reservoir zu befüllen.

Vorrichtungen zur Umfüllung von Arzneimitteln, meist unter Verwendung von so genannten Wegwerfspritzen, sind seit längerem bekannt. Im Wesentlichen wird dabei die Kanüle einer Wegwerfspritze durch eine Durchstechmembrane eines normalen Arzneimittelbehälters gestoßen und ein Arzneimittel aus dem Arzneimittelbehälter durch die Kanüle in die Wegwerfspritze aufgesogen.

Derselbe Umfüllvorgang liegt auch der Auffüllung einer Wegwerfampulle, welche in einer Infusionspumpe oder einem Injektionspen eingesetzt wird, zugrunde. Solche Infusionspumpen sind z.B. aus der EP 0 143 895 bekannt. Injektionspens sind z.B. aus der WO 87/02895 bekannt. Wegwerfampullen unterschieden sich von Wegwerfspritzen dadurch, dass der Stopfen nicht für einen manuellen Vorschub von Hand, sondern vielmehr zur Kopplung an eine elektromechanische Antriebsvorrichtung ausgelegt ist.

Die Befüllung der für den Einsatz in Infusionspumpen vorgesehenen Wegwerfampullen erfolgt in den meisten Fällen aus einem starren Vorratsbehältnis, welches am vorderen Ende mit einer Durchstechmembran versehen ist und im Vergleich zur Wegwerfampulle ein deutlich größeres Fassungsvermögen aufweist. Typisch sind etwa 10ml für das Vorratsbehältnis und 3 ml für die Wegwerfampulle. Der Vorgang einer sterilen und blasenfreien Umfüllung gestaltet sich jedoch für den Patienten vergleichsweise fehleranfällig und aufwändig. Deutlich einfacher gestaltet sich die Umfüllung aus einer so genannten Penampulle, welche eine dem Füllvolumen der Wegwerfampulle vergleichbare Medikamentenmenge beinhaltet. Die Penampulle ist am vorderen Ende mit einer Durchstechmembran und am hinteren Ende mit einem verschiebbaren Stopfen ausgestattet, wobei das Medikament vergleichbar einer Spritze durch Verschiebung des Stopfens aus dem zylindrischen Ampullenkörper verdrängt werden kann. Die Wegwerfampulle ist am vorderen Ende mit einer Durchstechmembran oder einem fluidischen Konnektor z.B. in Form eines männlichen Luer-Anschlusses ausgestattet. Am hinteren Ende ist das Medikamentenvolumen vergleichbar einer Spritze mit einem verschiebbaren Stopfen abgeschlossen, welcher beim Einsatz in der Infusionspumpe mit deren Antrieb gekoppelt wird.

Aus der DE 299 15 878 U1 ist eine Vorrichtung zur Umfüllung von Arzneimitteln mit einem Behälter bekannt, dessen rückseitige Öffnung durch einen beweglichen und mit einer Kolbenstange verbundenen Stopfen verschließbar ist und dessen vorderseitige Öffnung in einer ersten Kanüle mündet, wobei neben der ersten Kanüle eine zweite Kanüle angeordnet ist.

Aus der US 4,338,980 ist eine Vorrichtung bekannt, die zum Umfüllen eines Fluids aus einer Ampulle in einen Injektor geeignet ist. Die Ampulle und der Injektor sind über einen Stopfen verbunden, der beim Anpressen des Injektors an die Ampulle einen Verbindungskanal freigibt, um den Umfüllvorgang auszuführen.

Die Vorrichtung der WO 2004/032997 A2 ist zum Umfüllen einer Flüssigkeit aus einem Behälter in eine Ampulle geeignet. Hierzu wird die Ampulle mit einem Adapter verbunden, der anschließend mit dem Behälter über eine Verbindungsvorrichtung verbunden wird. Somit kann die Flüssigkeit aus dem Behälter über die Verbindungsvorrichtung in die Ampulle umgefüllt werden.

Die WO 03/090822 A1 offenbart eine Verbindungsvorrichtung, die eine erste Kammer mit einer zweiten Kammer verbindet. Diese Verbindungsvorrichtung weist eine doppelendige Nadel auf, die sowohl das Septum der ersten Kammer als auch das Septum der zweiten Kammer beim Zusammenschieben der Vorrichtung penetriert. Durch diese Verbindung kann ein Umfüllvorgang ausgeführt werden.

Aus der FR 2 762 990 ist eine Vorrichtung zum Umfüllen bekannt, in der eine Flüssigkeit aus einer Kammer über eine Düse in eine zweite Kammer umgefüllt wird.

US 6,162,199 offenbart eine klappbare Umfüllvorrichtung, in der eine Ampulle und eine Spritze aufgenommen werden können. Durch das Zusammenklappen der Umfüllvorrichtung wird die Verbindung zwischen der Ampulle und der Spritze hergestellt, so dass durch ein Aufziehen der Spritze die Flüssigkeit aus der Ampulle in die Spritze umgefüllt wird.

Aus der US 6,439,276 B 1 ist eine Umfüllvorrichtung bekannt, die entlang der Längsachse der Vorrichtung klappbar ist. Diese Vorrichtung weist eine erste Aufnahme zum Aufnehmen einer Spritze und eine zweite Aufnahme zum Aufnehmen einer Ampulle auf. Nach dem Einlegen der Spritze in die erste Aufnahme wird die Vorrichtung geschlossen und die Ampulle von der Stirnseite in die zweite Aufnahme eingeführt. Durch das Einführen penetriert die in der Vorrichtung befindlichen Spritze das Septum der Ampulle und erlaubt somit den Umfüllvorgang.

Es ist eine Aufgabe der vorliegenden Erfindung eine Vorrichtung zum Umfüllen einer Substanz oder eines Arzneimittels vorzuschlagen, welche von einem Benutzer einfach bedienbar ist und welche das Umfüllen von Arzneimitteln vereinfacht.

Diese Aufgabe wird durch eine Vorrichtung gemäß Anspruch 1 gelöst. Vorteilhafte Ausführungsformen sind in den abhängigen Ansprüchen definiert.

Eine Vorrichtung zum Umfüllen einer Substanz, wie zum Beispiel ein Arzneimittel oder Medikament, Insulin oder eine Hormone enthaltende Lösung, aus einem Vorratsbehälter, wie zum Beispiel einer Penampulle in eine Ampulle, welche in eine Pumpe eingesetzt werden kann, wie zum Beispiel eine Insulinpumpencartridge, weist einen Adapter oder eine Haltevorrichtung auf, um den Vorratsbehälter und die zu befüllende Ampulle relativ zueinander auszurichten und festzuhalten oder zu fixieren und zu koppeln. Dabei weist der Adapter vorzugsweise ein Führungselement oder eine Halterung für den Vorratsbehälter, also zum Beispiel die Pen-Ampulle, und für die zu befüllende Ampulle auf, so dass diese beiden Behälter oder Ampullen relativ zueinander ausgerichtet und vorzugsweise in der ausgerichteten Position fixiert werden können. Hierzu kann ein Führungselement des Adapters zum Beispiel als eine zum Teil über die Ampulle oder ein Ampullenende ragende Ummantelung ausgebildet sein. Es ist auch möglich den Adapter so auszulegen, dass die Ampullen relativ zueinander so ausgerichtet und bevorzugt fixiert oder festgehalten werden, dass nur an deren Eintritts- oder Austrittsöffnung eine Führung oder Halterung zum Ausrichten des jeweiligen Behälters oder der jeweiligen Ampulle vorgesehen ist. Des weiteren weist die Umfüllvorrichtung einen in den Vorrats- oder Abgabe-Behälter einschieb- oder einführbaren Verdrängungskörper oder Stößel auf, mit welchem zum Beispiel ein Stopfen in den Vorratsbehälter oder in die Vorrats-Ampulle eingeschoben werden kann.

Der erfindungsgemäße Adapter weist somit vorzugsweise Halterungen für den Vorratsbehälter, also zum Beispiel eine Pen-Ampulle, und den zu befüllenden Behälter, also zum Beispiel eine Pumpen-Ampulle auf, wobei vorteilhaft die Halterung so ausgebildet ist, dass mindestens 1/5 der axialen Länge des jeweiligen Behälters oder der jeweiligen Ampulle umfasst oder gehalten werden können. Allgemein soll unter dem Begriff "Adapter" im Sinne der Erfindung ein Element verstanden werden, welches die beiden Ampullen oder Behälter relativ zueinander ausrichtet oder positioniert oder hält.

Der Adapter kann auch so ausgebildet sein, dass durch den Adapter allein keine fluidische Kopplung der Behälter oder Ampullen realisiert wird und die Behälter oder Ampullen zum Beispiel durch entsprechende Führungsstege oder Führungsvorsprünge relativ zueinander in einer gewünschten Anordnung, zum Beispiel koaxial zueinander, ausgerichtet werden. Die Behälter oder Ampullen können dann zum Beispiel mittels einer an sich bekannten auf eine Ampulle aufsteckbaren Kanüle zur Übertragung der Substanz von einem Behälter in einen anderen gekoppelt werden.

Der Adapter zur lagerichtigen Ausrichtung der Ampullen ist als ein bevorzugt in Längsrichtung aufklappbares Element ausgebildet sein, in welches die Ampullen so eingelegt werden, dass die Öffnungen zum Beispiel einander gegenüberliegen. Dabei können die Ampullen schon fluidisch gekoppelt sein oder erst anschließend zum Beispiel mit einem Kopplungsstück gekoppelt werden. Anschließend kann der Adapter zugeklappt werden, um die Ampullen in ihrer eingelegten Lage zu fixieren und um zum Beispiel mit Hilfe eines Stößels die Fluid-Übertragung durchzuführen.

Um den Fluid-Übertragungsvorgang gleichmäßig ablaufen zu lassen, kann an dem Adapter ein Federelement vorgesehen sein, welches zum Beispiel durch Herausziehen des Stößels aufgezogen oder gespannt wird. Nachdem die Ampullen eingelegt und positioniert worden sind, kann der zum Beispiel in der herausgezogenen Stellung arretierte Stößel freigegeben oder losgelassen werden, so dass der Stößel durch die Kraft der vorgespannten Feder gleichmäßig in die Ampulle hineingedrückt wird, um so einen gleichmäßigen Übergang des Fluids in die zu befüllende Ampulle zu bewirken.

Zur gleichmäßigen Übertragung der Flüssigkeit oder des Fluides kann der Stößel auch mittels einer Gewindekopplung mit dem Adapter verbunden sein, so dass der Stößel in den Adapter eingeschraubt werden muss, um den Fluid-Übergang durchzuführen, wodurch eine ruckartige oder schnelle und ungleichmäßige Übertragung des Fluides verhindert wird.

Optional kann in einem Kopplungsstück zur Fluidübertragung eine Gas- oder Luftabscheidemembran vorgesehen sein, um zu verhindern, dass eine in der Ampulle mit der abzugebenden Substanz enthaltene Luft in die zu befüllende Ampulle übertragen wird. Luftabscheidende Membranen oder Luftfilter, welche gas- oder luftdurchlässig aber nicht fluiddurchlässig sind, sind im Stand der Technik bekannt.

Bevorzugt werden die Vorratsbehälter oder Ampullen so vorkonfektioniert ausgeliefert, dass auf jedem Vorratsbehälter oder jeder Ampulle schon ein Adapter aufgesetzt ist. Einem Satz von zum Beispiel zehn ausgelieferten Vorratsbehältern oder Ampullen mit jeweils aufgesetztem Adapter kann zum Beispiel ein einziger Stößel, welcher mehrfach verwendet werden kann, beigelegt werden.

Gemäß einer Ausführungsform weist der Adapter mindestens ein und bevorzugt zwei Rastelemente auf, um den Adapter mittels eines Rastelements mit dem Ampullenhalter zu verrasten und um den Adapter mittels eines weiteren Rastelements mit der zu befüllenden Ampulle zu verrasten, an welcher entsprechende Gegenrastelemente an der Abgabeöffnung vorgesehen sein können.

Der Adapter sollte aus hygienischen und regulatorischen Gründen als Einwegteil ausgebildet sein.

Die Erfindung wird nachfolgend anhand von Ausführungsbeispielen beschrieben. Es zeigen:
- Figur 1: die Anordnung der umzufüllenden Ampullen bei Verwendung einer Ausführungsform der Umfüllvorrichtung im Ausgangszustand;
- Figur 2: die Ampullenanordnung von Figur 1 im Endzustand;
- Figur 3: eine Ausführungsform einer aufklappbaren Umfüllvorrichtung;
- Figuren 4 und 5: Ausführungsformen zur Halterung der Ampullen,
- Figur 6: eine Detailansicht des hinteren Endes einer Umfüllvorrichtung;
- Figuren 7 bis 9: Beispiele des vorderen Endes einer Ampunenumfüllvorrichtung.

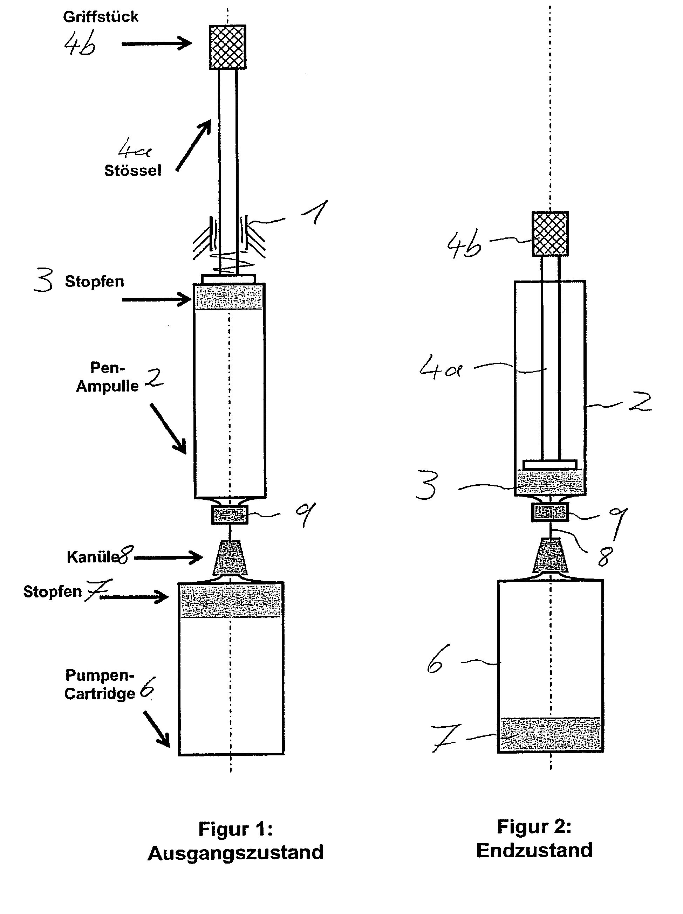

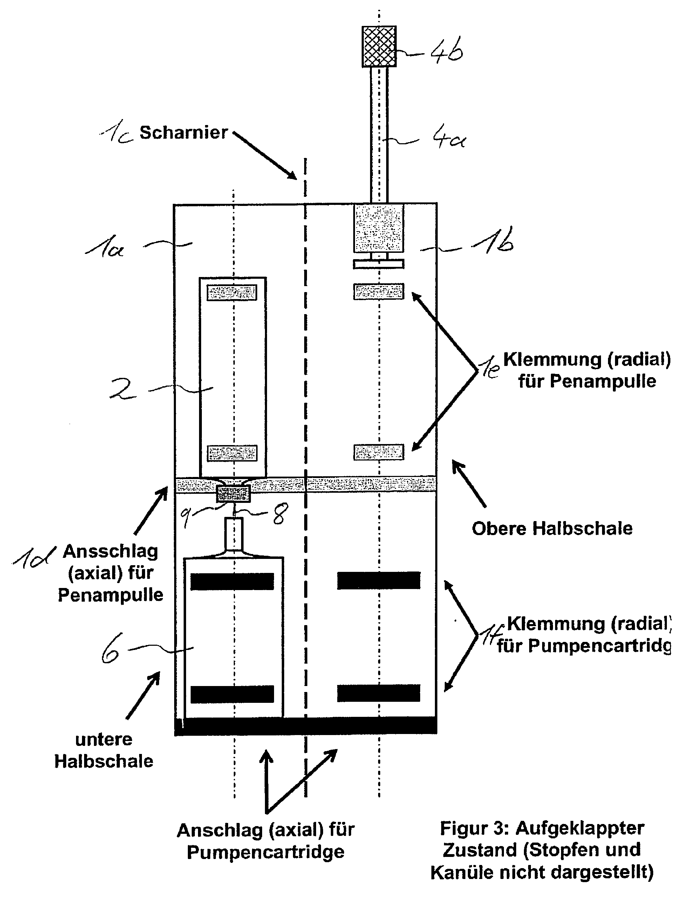

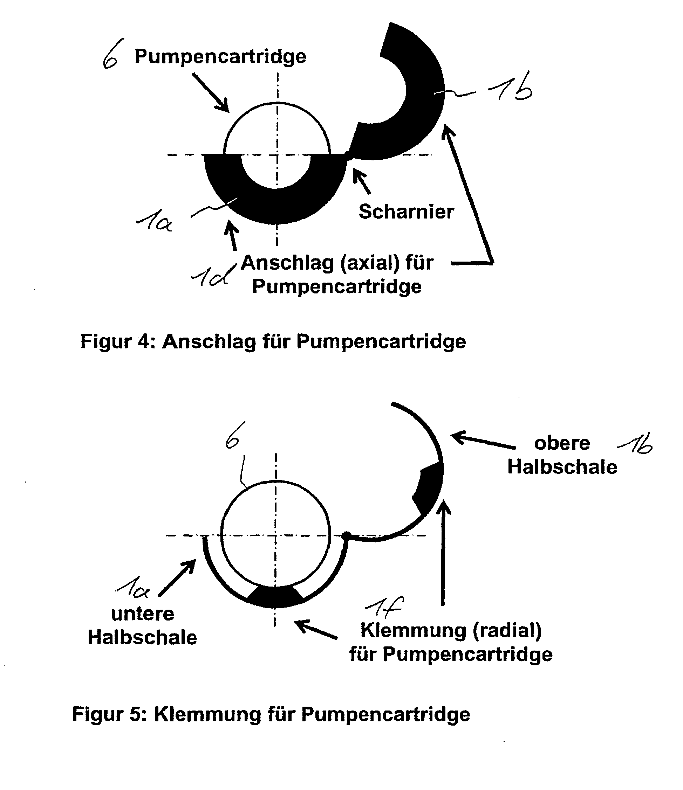

Figur 1 und Figur 2 zeigen die prinzipielle Anordnung der von einer Umfüllvorrichtung 1 gehaltenen Ampullen, wobei eine Pen-Ampulle 2 in eine Insulin-Ampulle oder Insulinpumpencartridge 6 umgefüllt werden soll, im Anfangs- und im Endzustand, d.h. vor Beginn und nach Beendigung des Umfüllvorgangs. Die nachfolgend anhand der Figur 3 beschriebene Umfüllvorrichtung hält die Ampullen 2, 6 in der gezeigten relativen Lage fest, nachdem zum Beispiel zunächst die leere Ampulle 6 mit einer an der Einlass- und Abgabeöffnung vorgesehenen Kanüle 8 und die gefüllte Ampulle 2 mit einem Septum 9 an der Abgabeöffnung eingesetzt wurde. Die Ampullen 2 und 6 sind in der gezeigten Ausführungsform koaxial zueinander angeordnet und werden zum Beispiel durch Halteelemente, Anschläge 1d und/oder Klemmungen If der Umfüllvorrichtung 1 in der gezeigten Anordnung gehalten, wie nachfolgend unter Bezug auf die Figuren 4 und 5 beschrieben.

Ein von einem Anwender betätigbarer Stößel 4a mit Griffstück 4b kann in die Umfüllvorrichtung 1 eingeschoben werden und drückt mit seiner Vorderseite auf einen in der Ampulle 2 verschiebbaren Stopfen 3, um so die Ampulle 2 zu entleeren und die in der Ampulle 2 enthaltene Substanz über die Fluidverbindung, welche durch die in das Septum 9 einstechende Kanüle 8 geschaffen wurde, in die zu befüllende Ampulle 6 zu übertragen, wobei der Stopfen 7 der Ampulle 6 während des Umfüllvorganges an das hintere Ende der Ampulle 6 geschoben wird, wie in Figur 2 gezeigt.

Nach erfolgtem Umfüllvorgang können die Ampullen 2 und 6 aus der Umfüllvorrichtung 1 entnommen werden.

Figur 3 zeigt in Draufsicht schematisch eine aufgeklappte Umfüllvorrichtung 1, in welche die Ampullen 2 und 6 eingelegt werden, nachdem die Fluid-Verbindung zwischen den Ampullen durch Einstecken der Kanüle 8 der Ampulle 6 in das Septum 9 der Ampulle 2 hergestellt wurde. Die Umfüllvorrichtung 1 besteht aus zwei zum Beispiel halbzylinderförmigen Schalen 1a und 1b, welche über ein Scharnier 1c miteinander klappbar verbunden sind, in welche axiale Anschläge 1d und radiale Klemmungen 1e, 1f zum Beispiel als Rippen zum Halten der Ampullen 2 und 6 im zusammengeklappten Zustand der Schalen 1a, 1b integriert sein können, Die Ampullen 2 und 6 werden im aufgeklappten Zustand in die Umfüllvorrichtung 1 eingelegt. Für den eigentlichen Umfüllvorgang werden die Schalen 1a und 1b von einem Benutzer zusammengeklappt, wobei der Klappvorgang in einer axialen Draufsicht in den Figuren 4 und 5 gezeigt ist, Die zusammengeklappten Schalen 1a und 1b können von einem Benutzer mit einer Hand zusammengehalten werden, wobei die Ampullen 2, 6 fixiert oder geklemmt werden, während mit der zweiten Hand der Stößel 4a zum Umfüllen der Ampullen in die Umfüllvorrichtung 1 eingeschoben werden kann.

Zur visuellen Überwachung des Umfüllvorganges können eine oder beide Halbschalen 1a, 1b der Umfüllvorrichtung 1 vollständig oder zum Teil aus einem transparenten Material bestehen. Ebenso können Ausschnitte oder Schlitze in einer oder beiden Halbschalen 1a, 1b vorgesehen sein, um eine optische Kontrolle des Umfüllvorganges zu ermöglichen.

Neben der gezeigten Anordnung sind weitere Varianten möglich, wie zum Beispiel eine Klemmung der Ampullen mit je drei oder mehr Klemmrippen 1f, die dann in einem Winkel von zum Beispiel je 120° zueinander angeordnet sind. Ferner kann das Zusammenhalten der Schalen 1a, 1b anstatt durch die Hand des Patienten auch zum Beispiel mit einem Rast- oder Schnappverschluss erfolgen.

Der Stößel 4a kann auch als Spindel mit Außengewinde ausgeführt werden, während die Stößelführung, die zum Beispiel Bestandteil der Umfüllvorrichtung 1 oder einer Halbschale 1a, 1b ist, ein Innengewinde enthält. In diesem Fall kann das Umfüllen sehr feinfühlig durch eine Drehbewegung des Stößels 4a erfolgen. Eine weitere Alternative besteht in einem Federantrieb, wobei zum Beispiel der Stößel 4a von einer gespannten Feder in die Umfüllvorrichtung 1 eingeschoben oder eingeschraubt wird.

Anstelle einer einfachen Kanüle 8 zur Kopplung der Ampullen 2, 6 kann auch ein Adapter oder Kopplungsteil mit integriertem Luflabscheider eingesetzt werden. Auf diese Weise wird die in der Penampulle 2 vorhandene Luft beim Umfüllvorgang automatisch entfernt Durch eine entsprechende mechanische Anordnung ist es auch möglich, eine Füllhilfe an die Pumpe zu koppeln und die Motorbewegung des Pumpenantriebs oder das motorische Zurückfahren der Pumpengewindestange zum Umfüllen zu nutzen.

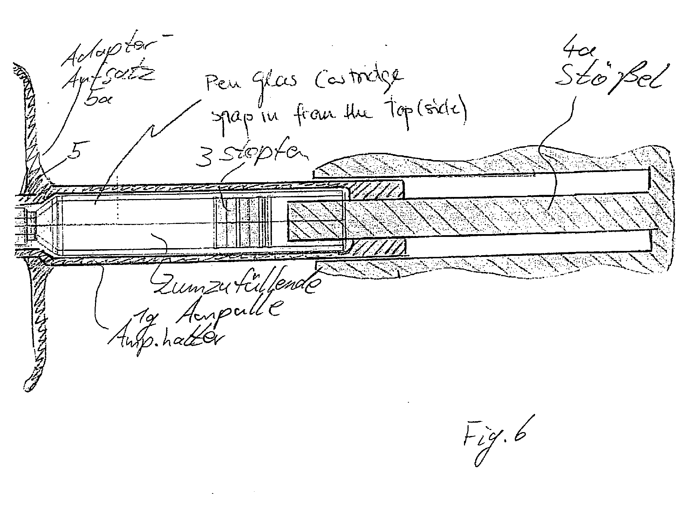

Figur 6 zeigt eine weitere Ausführungsform einer Umfüllvorrichtung 1, welche einen Ampullenhalter 1g aufweist, in welchen die Ampulle 2 mit der abzugebenden Substanz eingelegt wird.

Diese Ampulle 2 weist wiederum einen verschiebbaren Stopfen 3 auf, um die in der Ampulle 2 enthaltene Substanz zu verdrängen. Eine weitere Komponente ist ein Stößel 4a, welcher mit dem Ampullenhalter 1g gekoppelt ist und so auf den Ampullenhalter 1g aufgeschoben oder in diesen eingeschoben werden kann, dass ein an dem Stößel 4a angebrachter auf den Stopfen 3 wirkender Stab den Stopfen 3 beim Einschieben des Stößels 4a in den Ampullenhalter 1 in die Ampulle 2 einschiebt und so die Substanz aus der Ampulle 2 verdrängt bzw. abgibt. Als drittes Element ist ein Adapter 5 vorgesehen, auf welchen ein Adapteraufsatz 5a aufgesetzt wird. Der Adapter 5 koppelt die Abgabeöffnung oder den Ausgang der Ampulle 2 mit der Öffnung der zu befüllenden Ampulle 6, so dass während des Verschiebens des Stopfens 3 der Ampulle 2 mit der abzugebenden Substanz diese Substanz durch den Adapter 5 hindurch in die zu befüllende Ampulle 6 gelangen kann.

Prinzipiell ist es auch möglich, den Adapter 5 in den Ampullenhalter 1 zu integrieren, obwohl die Verwendung eines separaten Adapters 5 bevorzugt wird, da zum Beispiel eine an dem Adapter 5 vorgesehene in die zu befüllende Ampulle 6 und/oder in die zu entleerende Ampulle 2 einzustechende Nadel 8 aus hygienischen Gründen nur einmal verwendet werden sollte.

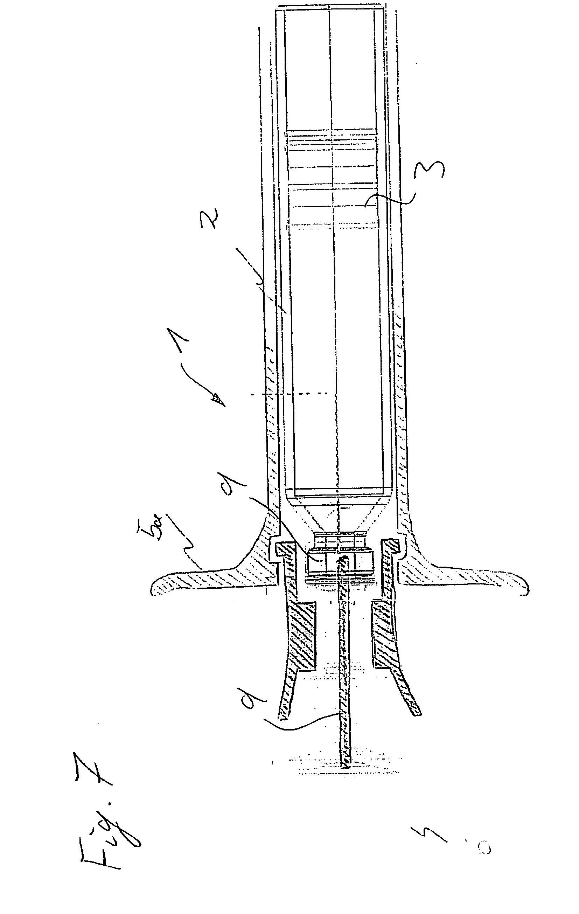

Die Figuren 7 bis 9 zeigen verschiedene Beispiele der Kopplung der zu befüllenden Ampulle 6 mit der Umfüllvorrichtung 1 oder der die einzufüllende Substanz enthaltenden Ampulle 2.

Figur 7 zeigt eine Umfüllvorrichtung 1, wobei die zu befüllende zum Beispiel aus Kunststoff oder Plastik bestehende Ampulle 6 fluidisch mit der Pen-Ampulle 2 mittels einer Kanüle 8 gekoppelt wird, welche mit der zu befüllenden Ampulle 6 verbunden ist und durch ein Septum 9 der Pen-Ampulle 2 eingestochen wird, um so eine Fluidverbindung zwischen dem Inneren der beiden Ampullen zu schaffen.

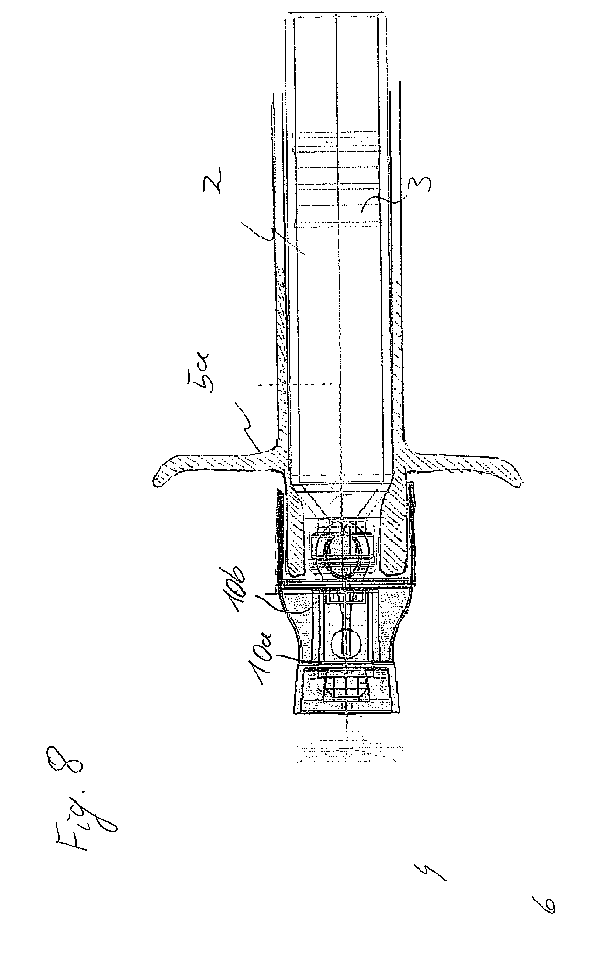

Figur 8 zeigt ein Beispiel einer Kopplung der Ampullen 2 und 6, wobei die zu befüllende Ampulle 6 einen Male-Luer-Verschluss 10a aufweist, welcher mit einem Female-Luer-Verschluss 10b der Pen-Ampulle 2 verbunden wird, um die Fluidverbindung zwischen den Ampullen 2 und 6 herzustellen.

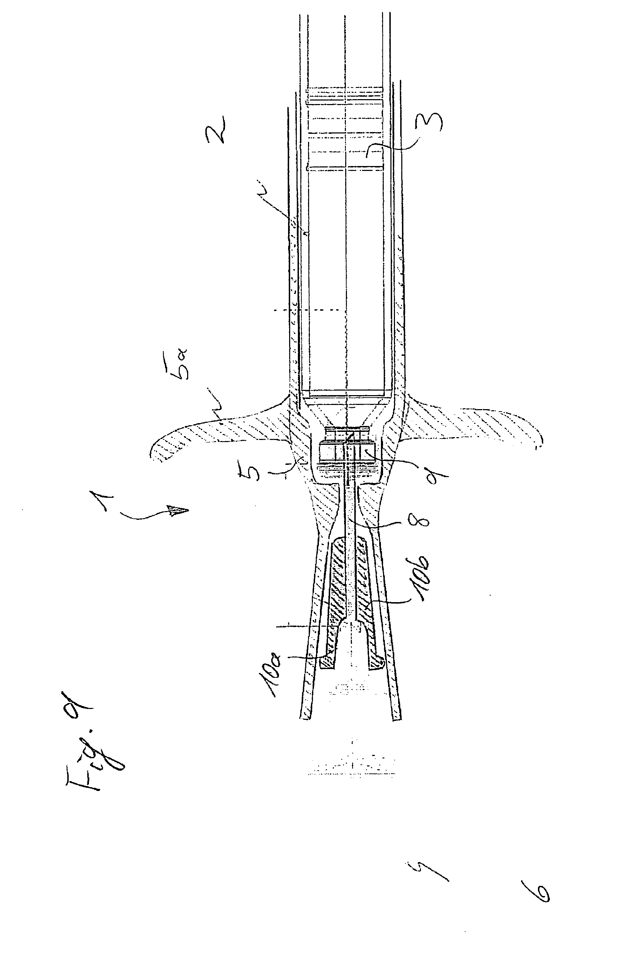

Figur 9 zeigt ein Beispiel einer Umfüllvorrichtung 1, bei welcher auf einen Male-Luer-Anschluss 10a der zu befüllenden Ampulle 6 ein Adapter 5 aufgesetzt ist, welcher aus einem Female-Luer-Anschluss 10b besteht, in welchen eine Kanüle 8 eingesetzt ist, mit welcher ein Septum 9 der Pen-Ampulle 2 durchstochen werden kann.

## Patentansprüche

1. Umfüllvorrichtung zum Umfüllen einer Substanz, insbesondere eines Arzneimittels, aus einem Vorratsbehälter (2) in eine Ampulle (6), welche in eine Vorrichtung zur dosierten Substanzabgabe eingesetzt werden kann,
a) mit einem Adapter (1, 5), um den Vorratsbehälter (2) und die Ampulle (6) relativ zueinander festzuhalten und zu koppeln, um eine in dem Vorratsbehälter (2) enthaltene Substanz in die Ampulle (6) umfüllen zu können und
b) mit einem klappbaren Element (1a, 1b), in welches in geöffnetem Zustand der Vorratsbehälter (2) und/oder die Ampulle (6) eingelegt werden kann und welches in zusammengeklapptem Zustand den Vorratsbehälter (2) und/oder die Ampulle (6) fixieren kann,
**gekennzeichnet durch**
c) ein Verdrängungselement (4a, 3), welches auf den Vorratsbehälter (2) einwirken kann, um die in dem Vorratsbehälter (2) enthaltende Substanz aus dem Vorratsbehälter (2) zu verdrängen und an die Ampulle (6) über die mittels des Adapters (5) realisierte Kopplung zu übertragen.

2. Umfüllvorrichtung nach Anspruch 1, wobei das Verdrängungselement ein Stößel (4a) oder ein in die Umfüllvorrichtung einschiebbares Element ist, welches auf den Vorratsbehälter (2) oder einen Stopfen (3) des Vorratsbehälters (2) einwirken kann, um den Vorratsbehälter (2) zu verformen und/oder den Stopfen (3) innerhalb des Vorratsbehälters (2) zu verschieben.

3. Umfüllvorrichtung nach einem der vorhergehenden Ansprüche, wobei der Adapter (5) einen Male-Luer-Anschluss (10a), einen Female-Luer-Anschluss (10b) und/oder eine Kanüle (8) aufweist, um die Anschlüsse oder Öffnungen des Vorratsbehälters (2) und der zu befüllenden Ampulle (6) zu koppeln.

4. Umfüllvorrichtung nach einem der vorhergehenden Ansprüche, wobei der Adapter (5) mindestens ein Führungselement (1d, 1f) aufweist, um den Vorratsbehälter (2) und/oder die zu befüllende Ampulle (6) relativ zum Adapter (5) und/oder relativ zueinander in einem definierten Lageverhältnis auszurichten.

5. Umfüllvorrichtung nach dem vorhergehenden Anspruch, wobei das Führungselement einen Ampullenhalter oder ein Rastelement aufweist.

6. Umfüllvorrichtung nach einem der vorhergehenden Ansprüche, wobei das Verdrängungselement oder der Stößel (4a) an oder in dem Adapter (1, 5) gerührt wird.

7. Umfüllvorrichtung nach einem der vorhergehenden Ansprüche mit mindestens einer oder zwei bevorzugt halbzylinderförmigen Schalen (1a, 1b), in welche der Vorratsbehälter (2) und/oder die Ampulle (6) eingelegt werden kann.

8. Umfüllvorrichtung nach einem der vorhergehenden Ansprüche, wobei die Umfüllvorriclitung und/oder der Adapter (5) ein durchsichtiges Material oder Sicht-Schlitze aufweist, um den Füllzustand des Vorratsbehälters (2) und/oder der Ampulle (6) erkennen zu können.

9. Umfüllvorrichtung nach einem der vorhergehenden Ansprüche, wobei der Stößel (4a) eine Spindel ist oder ein Gewinde aufweist oder durch einen Federantrieb angetrieben wird oder mit einem Motor einer Pumpe, insbesondere einer Vorrichtung zur dosierten Verabreichung einer Substanz, gekoppelt ist.

10. Umfüllvorrichtung nach einem der vorhergehenden Ansprüche, wobei der Adapter (5) oder ein Element im Bereich der Kopplung des Vorratsbehälters (2) mit der Ampulle (6) aus einem luftdurchlässigen Material ist, um in der Substanz enthaltenes Gas abzuscheiden.

11. System mit einer Umfüllvorrichtung nach einem der vorhergehenden Ansprüche und einem Vorratsbehälter (2) und/oder einer Ampulle (6).

## Claims

1. A transferring device for transferring a substance, in particular a medicine, from a storage container (2) into an ampoule (6) which can be inserted into a device for discharging a substance in doses, comprising:
a) an adaptor (1, 5) for securing the storage container (2) and the ampoule (6) relative to each other and coupling them in order that a substance contained in the storage container (2) can be transferred into the ampoule (6); and
b) a foldable element (1a, 1b) into which the storage container (2) and/or the ampoule (6) can be placed when it is open, and which can fix the storage container (2) and/or the ampoule (6) when it is folded closed,
**characterised by**
c) a displacement element (4a, 3) which can act on the storage container (2) in order for the substance contained in the storage container (2) to be displaced from the storage container (2) and transferred to the ampoule (6) via the coupling obtained by means of the adaptor (5).

2. The transferring device according to claim 1, wherein the displacement element is a ram (4a) or an element which can be pushed into the transferring device and which can act on the storage container (2) or a stopper (3) of the storage container (2) in order to deform the storage container (2) and/or to push the stopper (3) within the storage container (2).

3. The transferring device according to any one of the preceding claims, wherein the adaptor (5) comprises a male Luer connector (10a), a female Luer connector (10b) and/or a cannula (8) in order to couple the connectors or openings of the storage container (2) and the ampoule (6) to be filled.

4. The transferring device according to any one of the preceding claims, wherein the adaptor (5) comprises at least one guide element (1d, 1f) for orientating the storage container (2) and/or the ampoule (6) to be filled in a defined positional relationship relative to the adaptor (5) and/or relative to each other.

5. The transferring device according to the preceding claim, wherein the guide element comprises an ampoule holder or a catch element.

6. The transferring device according to any one of the preceding claims, wherein the displacement element or the ram (4a) is guided on or in the adaptor (1 ,5).

7. The transferring device according to any one of the preceding claims, comprising at least one or two preferably semi-cylindrical shells (1a, 1b) into which the storage container (2) and/or the ampoule (6) can be placed.

8. The transferring device according to any one of the preceding claims, wherein the transferring device and/or the adaptor (5) comprises a transparent material or viewing slits, in order to be able to identify the fill level in the storage container (2) and/or in the ampoule (6).

9. The transferring device according to any one of the preceding claims, wherein the ram (4a) is a spindle or comprises a thread or is driven by a spring drive or is coupled to a motor of a pump, in particular a device for administering a substance in doses.

10. The transferring device according to any one of the preceding claims, wherein the adaptor (5) or an element in the region of the coupling between the storage container (2) and the ampoule (6) is made of a material which is permeable to air, in order to separate off gas contained in the substance.

11. A system comprising a transferring device according to any one of the preceding claims and a storage container (2) and/or ampoule (6).

## Revendications

1. Dispositif destiné au transvasement d'une substance, en particulier d'un médicament, d'un réservoir de stockage (2) dans une ampoule (6), qui peut être inséré dans un dispositif servant à délivrer la substance de manière dosée,
a) avec un adaptateur (1, 5) pour retenir et coupler le réservoir de stockage (2) et l'ampoule (6) l'un par rapport à l'autre afin de pouvoir transvaser dans l'ampoule (6) une substance contenue dans le réservoir de stockage (2), et
b) avec un élément (1a, 1b) rabattable, dans lequel on peut introduire dans son état ouvert le réservoir de stockage (2) et/ou l'ampoule (6) et permettant à l'état rabattu de fixer le réservoir de stockage (2) et/ou l'ampoule (6),
**caractérisé par**
c) un élément de déplacement (4a, 3) qui peut agir au niveau du réservoir de stockage (2) afin de déplacer la substance contenue dans le réservoir de stockage (2) hors du réservoir de stockage (2) et la transmettre à l'ampoule (6) par le biais du couplage réalisé à l'aide de l'adaptateur (5).

2. Dispositif destiné au transvasement selon la revendication 1, l'élément de déplacement étant un coulisseau (4a) ou un élément pouvant être inséré dans le dispositif destiné au transvasement, qui peut agir sur le réservoir de stockage (2) ou un bouchon (3) du réservoir de stockage (2) afin de déformer le réservoir de stockage (2) et/ou de déplacer le bouchon (3) dans le réservoir de stockage (2).

3. Dispositif destiné au transvasement selon l'une quelconque des revendications précédentes, l'adaptateur (5) présentant un raccord luer mâle (10a), un raccord luer femelle (10b) et/ou une canule (8) afin de coupler les raccords ou ouvertures du réservoir de stockage (2) et de l'ampoule (6) à remplir.

4. Dispositif destiné au transvasement selon l'une quelconque des revendications précédentes, l'adaptateur (5) présentant au moins un élément de guidage (1d, 1f) afin d'orienter le réservoir de stockage (2) et/ou l'ampoule à remplir (6) par rapport à l'adaptateur (5) et/ou l'un par rapport à l'autre dans un rapport de position défini.

5. Dispositif destiné au transvasement selon la revendication précédente, l'élément de guidage présentant un support d'ampoule ou un élément d'encliquetage.

6. Dispositif destiné au transvasement selon l'une quelconque des revendications précédentes, l'élément de déplacement ou le coulisseau (4a) étant guidé sur ou dans l'adaptateur (1, 5).

7. Dispositif destiné au transvasement selon l'une quelconque des revendications précédentes avec au moins une ou deux coques (1a, 1b) de préférence semi-cylindriques, dans lesquelles le réservoir de stockage (2) et/ou l'ampoule (6) peut être introduite.

8. Dispositif destiné au transvasement selon l'une quelconque des revendications précédentes, le dispositif destiné au transvasement et/ou l'adaptateur (5) présentant un matériau transparent ou des fentes visibles afin de pouvoir reconnaître l'état de remplissage du réservoir de stockage (2) et/ou de l'ampoule (6).

9. Dispositif destiné au transvasement selon l'une quelconque des revendications précédentes, le coulisseau (4a) étant une broche ou présentant un filetage ou étant entraîné par un entraînement par ressort ou étant couplé à un moteur d'une pompe, en particulier d'un dispositif destiné à l'administration dosée d'une substance.

10. Dispositif destiné au transvasement selon l'une quelconque des revendications précédentes, l'adaptateur (5) ou un élément dans la zone du couplage du réservoir de stockage (2) à l'ampoule (6) étant en un matériau perméable à l'air afin d'extraire le gaz contenu dans la substance.

11. Système avec un dispositif destiné au transvasement selon l'une quelconque des revendications précédentes et un réservoir de stockage (2) et/ou une ampoule (6).
